# EUROPEAN PATENT SPECIFICATION

(11) **EP 4 735 410 B1**
(45) Date of publication and mention of the grant of the patent: **10.06.2026**
(21) Application number: 24751711.3
(22) Date of filing: 01.08.2024
(51) Int. Cl.: C07C 29/151, C07C 29/80, C07C 31/04

(54) **SYSTEM AND METHOD FOR THE PREPARATION OF BLUE METHANOL**
SYSTEM UND VERFAHREN ZUR HERSTELLUNG VON BLAUEM METHANOL
SYSTÈME ET PROCÉDÉ POUR LA PRÉPARATION DE MÉTHANOL BLEU

(30) Priority: 02.08.2023 DK PA202330140
(43) Date of publication of application: 06.05.2026
(73) Proprietor: Topsoe A/S, 2800 Kgs. Lyngby (DK)
(72) Inventor: ESKESEN, Søren, Grønborg, 2800 Kgs. Lyngby (DK); DAHL, Per, Juul, 2800 Kgs. Lyngby (DK); SHIM, Tine, 2800 Kgs. Lyngby (DK)
(74) Representative: Topsoe A/S
(86) International application number: PCT/EP2024/071906
(87) International publication number: WO 2025/027169

(56) References cited:
- WO-A1-2023/218160
- US-A1- 2020 109 108

## Description

### TECHNICAL FIELD

The present invention relates to a system and a process for producing blue methanol, where blue methanol is understood as methanol produced under conditions limiting the emission of CO₂. The system comprises a shift section, a CO₂ removal section and in a preferred embodiment also a hydrogen recovery section, arranged downstream a methanol synthesis section.

### BACKGROUND

Current methanol production system layouts and processes often involve a limited exploitation of by-products in the purge gas stream and/or flash gas stream issued as waste from the methanol synthesis production. In some instances, the purge gas is separated into a hydrogen-rich gas stream and an off-gas stream where the hydrogen-rich gas stream is reintroduced to the methanol synthesis production line and where the off-gas stream is fed to a fuel system as energy source, where the fuel system provides energy or heat elsewhere in the production.

US2020/109108 describes a process for producing formaldehyde-stabilized urea which comprises the preparation of methanol.

WO2023218160A1 describes a method for synthesis methanol with low emission of carbon dioxide.

However, current methanol production system layouts and processes result in the emission of flue gas to the atmosphere, which is associated with high CO₂ emission. In addition, current system layouts and processes result in an inefficient use of off-gas, which may still have a significant CH₄, CO, CO₂ and H₂ content. The high CO₂ emission and inefficient use of off-gas both goes against the current interests of the chemical industry where - in recent years - focus has been on reducing greenhouse gas emissions and increasing energy efficiency.

It is an object of embodiments of the invention to provide an effective system and process for methanol production in which at least a portion of the produced CO₂ can be captured, stored and/or used elsewhere. More specifically, the present technology provides a system and process where at least a portion of a flash gas stream and/or at least a portion of an off-gas stream is reintroduced to the methanol synthesis production line and used as an alternative or additional carbon comprising feed.

### SUMMARY

It has been found by the present inventors that the CO₂ emission from the methanol production processes can be significantly reduced if a shift section and a CO₂ removal section are introduced into the methanol production system layout and if at least a portion of gases isolated from the methanol production processes is reintroduced into the methanol synthesis production line. The provided system and process for producing methanol thereby increase the energy efficiency of the methanol production plant.

In a first aspect, therefore, a system is provided for producing methanol, said system comprising:
- a hydrocarbon feed;
- a steam feed;
- optionally, an oxygen feed;
- a reformer section (A) arranged to receive said hydrocarbon feed, said steam feed and, where present, said oxygen feed, and to provide a first synthesis gas stream;
- a cooling train (B), arranged to receive at least a portion of the first synthesis gas stream, and provide a second synthesis gas stream and a third synthesis gas stream;
- a methanol synthesis section (C) arranged to receive at least a portion of the second synthesis gas stream, optionally in admixture with a hydrogen-rich stream, and to provide a raw methanol stream, a first flash gas stream and a purge gas stream;
- a distillation section (D) arranged to receive at least a portion of the raw methanol stream and to provide a product methanol stream and an off-gas stream,
- a shift section (E) arranged to receive at least a portion of said purge gas stream, at least a portion of said third synthesis gas stream, optionally a steam feed, and provide a shifted gas stream;
- a CO₂ removal section (F) arranged to receive at least a portion of the shifted gas stream and provide a CO₂-rich gas stream and a first CO₂-depleted gas stream, and optionally a second flash gas stream;
wherein at least one of
i) at least a portion of the first flash gas stream from the methanol synthesis section (C),
ii) at least a portion of the off-gas stream from the distillation section (D),
is/are arranged to be fed as additional carbon comprising feed(s) to the reformer section (A), and/or to the shift section (E).

In a second aspect, a process for producing methanol in a system as described herein is also provided. The process comprises the steps of:
- feeding the hydrocarbon feed, the steam feed and, where present, the oxygen feed to the reformer section (A) to provide a first synthesis gas stream;
- feeding at least a portion of the first synthesis gas stream to the cooling train (B) to provide a second synthesis gas and a third synthesis gas;
- feeding at least a portion of the second synthesis gas stream, optionally in admixture with a hydrogen-rich stream, to a methanol synthesis section (C) and converting at least a portion of the second synthesis gas stream to a raw methanol stream, a first flash gas stream and a purge gas stream;
- feeding at least a portion of the raw methanol stream to a distillation section (D) to provide a product methanol stream and an off-gas stream;
- feeding at least a portion of the purge gas stream, at least a portion of the third synthesis gas stream, optionally a steam feed, to the shift section (E), to provide a shifted gas stream;
- feeding at least a portion of the shifted gas stream to a CO₂ removal section (F) to provide a CO₂-rich gas stream and a first CO₂-depleted gas stream, optionally a second flash gas stream;
- and further feeding at least one of
   i) at least a portion of the first flash gas stream from the methanol synthesis section (C),
   ii) at least a portion of the off-gas stream from the distillation section (D),
to the reformer section (A), and/or to the shift section (E) as additional carbon comprising feed(s).

A plant is also provided which comprises the system as described herein.

Further details of the system and process for producing blue methanol are specified in the following detailed description, figures and claims.

### LEGENDS

Figure 1 is a schematic illustration of a general methanol production layout, this figure serves as an example of the prior art.
Figure 2 is a schematic illustration of the invention in a first embodiment, where a shift section (E) and a CO₂ removal section (F) are incorporated in the methanol production system layout and at least one of i) a portion of a first flash gas stream from the methanol synthesis section and/or ii) a portion of an off-gas stream from the distillation section is/are arranged to be fed as additional carbon comprising feed(s) to the reformer section (A), and/or to the shift section (E).
Figure 3 is a schematic illustration of the invention in a second embodiment, a portion of the second flash gas stream from the CO₂ removal section (F) is sent to the reformer section (A) as an additional carbon comprising feed.
Figure 4 is a schematic illustration of the invention in a third embodiment, where a portion of the CO₂-depleted gas stream from the CO₂ removal section (F) is sent to the methanol synthesis section (C), preferably in admixture with the second synthesis gas stream.
Figure 5 is a schematic illustration of the invention in a fourth embodiment, where the system further comprises a hydrogen recovery section (G) and a common fuel system (H). The hydrogen recovery section (G) provides a first portion of a first off-gas stream to the reformer section (A) as an additional carbon comprising feed.

### DETAILED DISCLOSURE

Unless otherwise specified, any given percentages for gas content are % by volume. The terms "synthesis gas" and "syngas" are used interchangeably in this text.

A system for producing methanol is provided which comprises arrangements of feeds and sections. For the avoidance of doubt, the term "feed" refers to means for supplying said gas to the appropriate section; and the term "section" refers to one or more reactors or units where in a transformation of gas e.g., reaction, pressure change, temperature change etc. can occur.

More specifically, a system is provided for producing methanol. The system comprises - in general terms - a hydrocarbon feed, a steam feed and optionally, an oxygen feed, a reformer section (A); a cooling train (B); a methanol synthesis section (C); a distillation section (D); a shift section (E); and a CO₂ removal section (F). These feeds and sections will be described in more detail in the following.

The feeds and sections are arranged in such a way, that the total carbon emission from the methanol production plant is significantly reduced, in part because the systems allow for carbon capture and storage, wherein the carbon is in the form of CO₂ (g) and specifically because the systems allow for keeping carbon containing compounds within the system. More specifically, carbon containing compounds are kept as carbon comprising streams and is/are re-introduced into the system as additional carbon comprising feed(s). The arrangement further saves on incoming natural gas as feedstock and makes room for using hydrogen rich fuel gas instead of natural gas or alternative hydrocarbon feed, such to decrease carbon emission. The arrangement of the system has the potential for a CO₂ reduction of more than 97%, such as above 99% CO₂ reduction relative to the sum of carbon content in the hydrocarbon feed fed to the reformer section (A) and hydrocarbon fuel provided for production.

### Hydrocarbon feed

The hydrocarbon feed serves as the carbon source for the methanol production plant. The hydrocarbon feed can be any hydrocarbon feed such as natural gas or a purified hydrocarbon feed. In a preferred embodiment the hydrocarbon feed is a purified hydrocarbon feed. In embodiments, the purified hydrocarbon feed is purified from e.g., sulphur. A purified hydrocarbon feed is suitably "hydrocarbon rich" meaning that the major portion of this feed is hydrocarbons; i.e., over 50%, e.g., over 75%, such as over 85%, preferably over 90%, more preferably over 95%, even more preferably over 99% of this feed is hydrocarbons.

### Reformer section

The reformer section (A) is arranged to receive the hydrocarbon feed and, dependent on the applied reformer technology, optionally also, a steam feed and - where present - an oxygen feed. The reformer section (A) converts the hydrocarbon feed, the steam feed and, where present, oxygen feed to a first synthesis gas stream.

In preferred embodiments, the reformer section (A) may be arranged to receive additional carbon comprising feed(s) re-directed from other sections comprised in the system. More specifically, the reformer section may further be arranged to receive at least one of i) at least a portion of the first flash gas stream from the methanol synthesis section (C), ii) at least a portion of the off-gas stream from the distillation section (D) as additional carbon comprising feed(s). Additionally, or alternatively, the reformer section may be arranged to receive iii) at least a portion of the second flash gas stream from the CO₂ removal section (F). Hence, the reformer section (A) may convert the hydrocarbon feed, the steam feed and, where present, oxygen feed and one or more of i)-iii) to a first synthesis gas stream.

The first synthesis gas stream provided comprises a high content of CO, CO₂ and H₂ and can further comprise CH₄, N₂, and Ar. Accordingly, the first synthesis gas stream provides the components for methanol synthesis.

The first synthesis gas stream suitably has the following dry gas composition by volume: 60-70% H₂, 3-10% CO₂, 20-30% CO, 0.5-4% CH₄ and other hydrocarbons. The first synthesis gas stream suitably has the following water content by volume: 14-45% H₂O.

The reformer section (A) may comprise one or more prereformer(s), steam reformer(s) and autothermal reformer(s) or any two combinations thereof. In a preferred embodiment the reformer section (A) comprises an adiabatic prereformer. In another preferred embodiment the reformer section (A) comprises an autothermal reformer or a tubular reformer followed by an autothermal reformer or a tubular reformer. The skilled person may select suitable arrangements of reformers, depending on the feeds available and the output required.

In a preferred embodiment, a purified hydrocarbon feed is mixed with a steam feed to obtain a molar steam/carbon ratio between 0.35 and 2.0 preferably between 0.5 and 1.5.

In an alternative preferred embodiment, a purified hydrocarbon feed is mixed with a steam feed and with at least one of i) at least a portion of the first flash gas stream from the methanol synthesis section (C), ii) at least a portion of the off-gas stream from the distillation section (D) and/or iii) at least a portion of the second flash gas stream from the CO₂ removal section (F) to obtain a molar steam/carbon ratio between 0.35 and 2.0.

The purified hydrocarbon feed and steam feed admixture, optionally also comprising one or more of i), ii) and/or iii), is sent through a prereforming reactor where hydrocarbons except for CH₄ are reduced to less than 0.5 mol %, preferably less than 0.2 mol% thus providing a prereformed gas stream. The prereformed gas stream is optionally sent through a heated tubular reformer for further conversion of CH₄ to CO, CO₂ and H₂, thereby providing a tubular reformed gas stream. The prereformed gas or optionally the tubular reformed gas is sent through an autothermal reformer where an oxygen feed with a purity higher than 90 mol%, preferably higher than 98 mol% is also added for further reforming of CH₄. In this way, the reformer section provides the first synthesis gas stream.

### Cooling train

A cooling train (B) is arranged to receive at least a portion of the first synthesis gas stream. The cooling train (B) is arranged to provide sensible heat to produce steam, superheat steam, re-boiling duty for distillation and water preheat. The cooling train (B) gradually cools the first synthesis gas. The cooling train (B) can further be arranged to allow for separation of condensed H₂O. In all embodiments, the cooling train (B) is arranged to provide a second synthesis gas stream and a third synthesis gas stream.

In a preferred aspect, the cooling train (B) is arranged to provide the third synthesis gas stream at a higher temperature than the second synthesis gas stream. The temperature of the second synthesis gas stream is typically between 20 and 70°C, preferably as low as possible depending on the available cooling media, which typically depends on the ambient air conditions.

The temperature of the third synthesis gas stream is preferably between 10°C above the dew point of the third synthesis gas stream and 360°C. The temperature of the third synthesis gas stream varies with the specific technology applied in the shift section, but is preferably between 140 and 340°C, more preferably between 145 and 240°C.

In a most preferred embodiment, the composition of the second synthesis gas stream is identical to the third synthesis gas stream. However, the third synthesis gas stream may differ from the second synthesis gas stream only in terms of the H₂O content. In the second synthesis gas stream, the water content may be less than 1 % preferably less than 0.25 %. The separated water will contain small amounts of dissolved gases, which has an insignificant effect on the composition of the second and third synthesis gas stream.

### Methanol synthesis section

The methanol synthesis section (C) is arranged to receive at least a portion of the second synthesis gas stream. The methanol synthesis section (C) converts the synthesis gas stream to a raw methanol stream, a first flash gas stream and a purge gas stream. The methanol synthesis reactor in the methanol synthesis section accommodates the following two reactions:

CO₂ + H₂ ⇆ CO + H₂O (1)

and

CO + 2H₂ ⇆ CH₃OH (2).

The process of converting the second synthesis gas stream can occur, for example by compressing the second synthesis gas stream and sending the compressed gas through a boiling water reactor, where at least a portion of the CO, CO₂ and H₂ is converted to methanol followed by a condensation section separating a liquid raw methanol stream from unconverted synthesis gas. A purge gas stream is withdrawn containing inerts such as methane. The liquid raw methanol stream is depressurized to around 4 bar g thereby producing a first flash gas stream containing primarily CO₂, secondarily H₂ and CH₄.

The raw methanol stream comprises a major portion of methanol; i.e., over 75%, such as over 85%, preferably over 90%, more preferably over 95% of this feed is methanol on weight basis. Other minor components of this stream include but not limited to, higher alcohols, ketones, aldehydes, dimethyl ether (DME), organic acids and dissolved gases.

To obtain an optimized yield in the methanol production, the stoichiometry of H₂, CO and CO₂ need to be considered. In a preferred embodiment, the stoichiometry of H₂, CO and CO₂ in the second synthesis gas stream falls within an interval such that the second synthesis gas stream has a module between 1.8 and 2.2, preferably between 1.9 and 2.1, where the module is defined in terms of molar content:

M = (H₂-CO₂)/(CO+CO₂).

The module of the second synthesis gas stream may be adjusted by addition of a hydrogen-rich stream, which is optionally arranged to be admixed with the second synthesis gas stream. The hydrogen-rich stream can be provided by an external feed of hydrogen, however in a preferred embodiment the hydrogen-rich stream is provided by other streams isolated downstream the methanol synthesis section (C).

The purge gas stream withdrawn from the methanol synthesis section typically comprises 50-70% H₂, 20-30% inerts (such as CH₄, N₂, Ar), 0-10% CO, and 0-10% CO₂. The pressure of the purge gas stream is typically high such as 70 - 100 bar g, preferably 80-90 bar g.

Typically, the first flash gas stream comprises 40-50% CO₂, 10-30% H₂, 0-10% CO, the rest being CH₄ and other inert gasses such as N₂ and Ar, and methanol (volume basis). The pressure of the first flash gas stream is typically below 10 bar g, such as 2-6 bar g., preferably around 4 bar g.

### Distillation section

A distillation section (D) is arranged to receive at least a portion of the raw methanol stream and to provide a product methanol stream and an off-gas stream.

The distillation section (D) upgrades the raw methanol stream to a purified methanol stream of the required grade, e.g., >95%, >98% or >99% methanol by weight.

In preferred embodiments, at least a portion of said off-gas stream from the distillation section (D) is arranged to be fed as an additional carbon comprising feed to the reformer section (A), and/or to the shift section (E). The off-gas stream comprises 70-90% CO₂, 0-15% methanol, 0-10% CH₄ and other inert gases such as N₂ and Ar as well as 0-10% higher alcohols, ketones, aldehydes, and/or dimethyl ether (DME). Typically, the pressure of the off-gas stream is between 0.0 and 1.0 bar g such as preferably close to atmospheric pressure of 0 bar g.

Specifically for the system is the re-direction of streams comprising carbon containing compounds such that said streams is/are reintroduced into the methanol synthesis production line. More specifically, in a first aspect, at least one of i) at least a portion of the first flash gas stream from the methanol synthesis section (C), ii) at least a portion of the off-gas stream from the distillation section (D), is/are arranged to be fed as additional carbon comprising feed(s) to the reformer section (A), and/or to the shift section (E). In a specifically preferred embodiment, substantially all of the first flash gas stream from the methanol synthesis section (C), and substantially all of the off-gas stream from the distillation section (D), is/are arranged to be fed as additional carbon comprising feed(s) to the reformer section (A), and/or to the shift section (E).

### Shift section

The shift section (E) is arranged to receive at least a portion of the purge gas stream, at least a portion of the third synthesis gas stream provided by the cooling train (B) optionally a steam feed and provide a shifted gas stream from these streams. In preferred embodiments said shift section may further be arranged to receive at least one of i) at least a portion of the first flash gas stream from the methanol synthesis section (C), ii) at least a portion of the off-gas stream from the distillation section (D) as additional carbon comprising feed(s) and provide a shifted gas stream from the streams received by said shift section (E).

The shift section (E) shifts the CO to CO₂ in accordance with the shift reaction:

H₂O (g) + CO (g) ⇆ CO₂ (g) + H₂ (9) (3),

such that the provided shifted gas stream is rich in CO₂ whereas the CO content is minimized to preferably less than 2%, more preferably less than 1% on dry gas basis.

In a preferred embodiment the molar steam/dry gas ratio at the shift section (E) inlet is in the range from 0.1 to 1.0, preferably 0.3 to 0.8. This is to ensure optimal conversion of CO to CO₂. Depending on the gas composition at the inlet, an additional steam feed is added to obtain the optimal steam/dry gas ratio. In a preferred embodiment the steam feed is arranged to be mixed with the purge gas stream and/or the third synthesis gas stream and/or the first flash gas stream and/or the off-gas stream from said distillation section (D) prior to being inlet to the shift section (E). In another preferred embodiment the steam feed is arranged to be fed to the shift section (E) as a separate feed.

The shift section (E) comprises a high temperature (HT) shift reactor, a medium temperature (MT) shift reactor or a low temperature (LT) shift reactor, or any combination of two or more of these. In preferred embodiments, the shift section (E) comprises one or more shift reactors. Within these embodiments the shift section (E) may comprise one or more adiabatic reactor(s). In preferred embodiments, the one or more HT shift reactor(s) operates with an inlet temperature between 300°C and 360°C, the one or more MT shift reactor(s) operates with an inlet temperature of 200°C to 280°C and the one or more LT shift reactor(s) operates with an inlet temperature of 180°C to 250°C. Alternatively, the shift section comprises one or more boiling water reactor(s), which allows for pushing the shift reaction further compared to HT shift reactor(s) as the temperature is kept down such as to a temperature of below 290°C.

The temperature in the shift section (E) is at least in part controlled by the temperature of the third synthesis gas stream provided from the cooling train (B) to the shift section (E). The cooling train (B) is arranged to provide the third synthesis gas stream at a temperature between 10°C above the dew point of the third synthesis gas stream and 360°C, dependent on the reactor or reactor combination comprised in the shift section (E). Typically, the dew point of the third synthesis gas stream is between 130-180°C.

In some embodiments the pressure of the third synthesis gas stream is increased upstream to the shift section. In a preferred embodiment the pressure of the third synthesis gas stream is increased by 2 to 6 bar prior to the inlet of the shift section (E). Where the gas needs to be compressed, i.e., between the cooling step and shift section, the temperature of the third synthesis gas stream will be between 10°C above the dew point of the third synthesis gas stream and 200°C. Suitably, the third synthesis gas stream has a temperature between 150°C and 340°C.

### CO₂ Removal section

The CO₂ removal section (F) is arranged to receive at least a portion of the shifted gas stream (and, optionally, the entirety of the shifted gas stream) and provide a CO₂-rich gas stream, a first CO₂-depleted gas stream and optionally a second flash gas stream therefrom. In this way, the CO₂-rich gas stream can be sent to storage or supplied for other purposes.

In a preferred embodiment, the CO₂-rich gas stream comprises >90% vol. CO₂, such as > 98% vol. CO₂, preferably > 99% vol. CO₂. In a preferred embodiment, the first CO₂-depleted gas stream has a CO₂ content below 1000 ppm vol., preferably below 500 ppm vol., more preferably below 20 ppm vol..

The pressure of the second flash gas stream is typically below 10 bar g. such as 3-7 bar g., preferably around 5 bar g. The composition of said second flash gas stream may comprise 60-70% CO₂ and 30-40% H₂ volume basis. In a preferred embodiment, at least a portion of the second flash gas stream from the CO₂ removal section (F), is arranged to be fed to the reformer section (A) as an additional carbon comprising feed. In this way, the second flash gas stream comprising carbon containing compounds is reintroduced into the methanol synthesis production line.

The CO₂ removal section (F) may comprise any conventional CO₂ removal technology, for example but not limited to an amine wash, or cold box solution.

Embodiments of the invention provide a system for methanol synthesis, where it follows from the feeds, sections, and the arrangement thereof that CO₂ is captured and can be stored and further those streams comprising carbon containing compounds (previously used as fuel feeds) is/are now kept within the system and used as additional carbon feed(s). The effect of the system is a significant reduction in emitted CO₂ due to methanol production. Embodiments can ensure that the carbon content in the combined flue gases (i.e., from burning hydrogen-rich gas and hydrocarbon fuel) is less than 3% of the sum of carbon content in the hydrocarbon feed fed to the reformer section and hydrocarbon fuel provided for production.

A plant comprising the system above is also provided.

### Specific embodiments

In embodiments of the system, at least one of
- at least a portion of the first flash gas stream from the methanol synthesis section (C),
- at least a portion of the off-gas stream from the distillation section (D), is/are arranged to be fed as additional carbon comprising feed(s) to the reformer section (A), and/or to the shift section (E).

In a preferred embodiment of the system at least a portion of the first flash gas stream from the methanol synthesis section (C) or at least a portion of the off-gas stream from the distillation section (D) is arranged to be fed to the reformer section (A) or the shift section (E).

In a preferred embodiment of the system at least a portion of the first flash gas stream from the methanol synthesis section (C) is arranged to be fed to the reformer section (A) and at least a portion of the off-gas stream from the distillation section (D) is arranged to be fed to the shift section (E).

In another preferred embodiment of the system at least a portion of the first flash gas stream from the methanol synthesis section (C) is arranged to be fed to the shift section (E) and at least a portion of the off-gas stream from the distillation section (D) is arranged to be fed to the shift section (A).

In alternative preferred embodiments of the system both at least a portion of the first flash gas stream from the methanol synthesis section (C) and at least a portion of the off-gas stream from the distillation section (D) is arranged to be fed to the reformer section (A) or both at least a portion of the first flash gas stream from the methanol synthesis section (C) and at least a portion of the off-gas stream from the distillation section (D) is arranged to be fed to the shift section (E).

In embodiments of the system, at least one of
- substantially all of the first flash gas stream from the methanol synthesis section (C),
- substantially all of the off-gas stream from the distillation section (D), is/are arranged to be fed as additional carbon comprising feed(s) to the reformer section (A), and/or to the shift section (E).

In a preferred embodiment of the system substantially all of the first flash gas stream from the methanol synthesis section (C) or substantially all of the off-gas stream from the distillation section (D) is arranged to be fed to the reformer section (A) or the shift section (E).

In preferred embodiments of the system, substantially all of the first flash gas stream from the methanol synthesis section (C), and substantially all of the off-gas stream from the distillation section (D), is/are arranged to be fed as additional carbon comprising feed(s) to the reformer section (A), and/or to the shift section (E).

In a preferred embodiment of the system substantially all of the first flash gas stream from the methanol synthesis section (C) is arranged to be fed to the reformer section (A) and substantially all of the off-gas stream from the distillation section (D) is arranged to be fed to the shift section (E).

In another preferred embodiment of the system substantially all of the first flash gas stream from the methanol synthesis section (C) is arranged to be fed to the shift section (E) and substantially all of the off-gas stream from the distillation section (D) is arranged to be fed to the shift section (A).

In alternative preferred embodiments of the system both substantially all of the first flash gas stream from the methanol synthesis section (C) and substantially all of the off-gas stream from the distillation section (D) is arranged to be fed to the reformer section (A) or both substantially all of the first flash gas stream from the methanol synthesis section (C) and substantially all of the off-gas stream from the distillation section (D) is arranged to be fed to the shift section (E).

In a preferred embodiment, the system further comprises that at least a portion of the second flash gas stream from the CO₂ removal section (F), is arranged to be fed to the reformer section (A) as an additional carbon comprising feed. In this way an additional carbon comprising stream is recycled into the methanol production line, further saving on incoming natural gas as feedstock, making room for using hydrogen rich fuel gas instead such to decrease carbon emission.

In preferred embodiments, the system further comprises the shift section (E) comprising one or more shift reactors. In a preferred embodiment, said shift section (E) comprises two or more shift reactors. As the shift section shifts the CO to CO₂ in accordance with the shift reaction, embodiments comprising two or more shift reactors results in an increased capacity for shifting the received stream(s) compared to a shift section comprising one shift reactor. Said capacity increases with increasing numbers of shift reactors comprised in said shift section (E).

In a preferred embodiment, the system further comprises a hydrogen recovery section (G). The hydrogen recovery section (G) is arranged to receive at least a portion of the first CO₂-depleted gas stream from the CO₂ removal section (F), and to provide at least a first hydrogen-rich stream, and at least a first off-gas stream.

The hydrogen recovery section (G) can utilise several technologies for separation of gases. Thus, the hydrogen recovery section can comprise a pressure swing adsorption unit, or a membrane. Independently of the technology applied, the provided first hydrogen-rich stream comprises primarily hydrogen preferably more than 80% more preferably more than 90%.

The first off-gas stream from this hydrogen recovery section comprises 15 to 45% CH₄, 0 to 20% N₂ and 0 to 4% Ar.

In this embodiment, comprising the hydrogen recovery section, the system can be arranged to provide at least a first portion of the first off-gas stream to the reformer section (A) as an additional carbon comprising feed. This arrangement makes effective use of hydrocarbon-containing feeds in the system.

Alternatively, or in additionally, in this embodiment, the system may be arranged to provide at least a first portion of the hydrogen-rich stream to the methanol synthesis section (C), preferably in admixture with the second synthesis gas stream. This arrangement makes effective use of hydrogen-containing streams and limits the need for an external hydrogen feed for regulating of the second synthesis gas stream module.

As an additional arrangement of this embodiment, the system can be arranged to further comprise a fuel system (H) and such that said hydrogen recovery section (G) system is arranged to provide a second portion (15b) of the first off-gas stream (15) and/or a second portion (14c) of the hydrogen-rich stream (14), wherein at least a portion of said second portion (15b) of the first off-gas stream (15) and/or at least a portion of said second portion (14c) of the hydrogen-rich stream (14) is arranged to be fed as a fuel feed to said fuel system (H) and/or an auxiliary steam boiler and/or a gas turbine. In addition, the fuel system is fed with a hydrocarbon feed. The fuel system provides the energy necessary for the methanol production. In this embodiment, the combusted gas stream from the fuel system is emitted to the atmosphere as flue gas. In this way, the purge gas, and at least one of a portion of the first flash gas and/or a portion of the off-gas from the methanol synthesis production constitutes an energy source necessary for the methanol synthesis process, and the requirement of external fuel sources can be reduced.

Alternatively, or in additionally, in this embodiment, the system further comprising a fuel system (H), at least a portion of the first CO₂-depleted gas stream provided from the CO₂ removal section may be arranged to be split into at least a second CO₂-depleted gas stream and at least a third CO₂-depleted gas stream. In this embodiment, at least a portion of said second CO₂-depleted gas stream can be arranged to be fed as a fuel feed to said fuel system (H). Also in this embodiment, the purge gas, and at least one of a portion of the first flash gas and/or a portion of the off-gases from the methanol synthesis production constitutes an energy source necessary for the methanol synthesis process, and the requirement of external fuel sources can be reduced.

In an alternative preferred embodiment, the principle of the invention can be used to achieve a smaller reduction of CO₂ emissions. This can for example be relevant in refurbishment of existing plants.

In this embodiment, the invention can be performed without the hydrogen recovery section (G). Thus, at least a portion of the first CO₂-depleted gas stream provided by the CO₂ removal section (F) is arranged to be split into at least a second CO₂-depleted gas stream and at least a third CO₂-depleted gas stream. Both the second and third CO₂-depleted gas streams are rich in CH₄ and H₂ and comprise N₂ and Ar.

In this alternative embodiment, at least a portion of said second CO₂-depleted gas stream may be arranged to be provided to the methanol synthesis section (C), preferably in admixture with the second synthesis gas stream.

With this arrangement, the second CO₂-depleted gas stream may constitute a source of H₂ used to adjust the equilibrium of species i.e., the module in the synthesis gas stream upstream the methanol synthesis section. The second CO₂-depleted gas stream is thus an alternative to the hydrogen-rich stream used in earlier embodiments. The third CO₂-depleted gas stream is used as fuel.

The carbon content of the third CO₂-depleted gas stream can to a lesser extent be controlled by the flow of the third synthesis gas stream, the limit being decided by the CH₄ content in the first synthesis gas stream, as all the CH₄ still present in the third CO₂-depleted gas stream will be used as fuel. This is as opposed to other specific embodiments of the invention where part of the CH₄ still present in second CO₂-depleted gas stream is used as alternative hydrocarbon feed in reformer section (A).

A plant comprising any one of the specific embodiments above is also provided.

A process for producing methanol in the system described herein is also provided. This process comprises the steps of:
- feeding the hydrocarbon feed, the steam feed and, where present, the oxygen feed to the reformer section (A) to provide a first synthesis gas stream;
- feeding at least a portion of the first synthesis gas stream to the cooling train (B) to provide a second synthesis gas and a third synthesis gas;
- feeding at least a portion of the second synthesis gas stream, optionally in admixture with a hydrogen-rich stream, to a methanol synthesis section (C) and converting at least a portion of the second synthesis gas stream to a raw methanol stream, a first flash gas stream and a purge gas stream;
- feeding at least a portion of the raw methanol stream to a distillation section (D) to provide a product methanol stream and an off-gas stream;
- feeding at least a portion of the purge gas stream, at least a portion of the third synthesis gas stream, optionally a steam feed, to the shift section (E), to provide a shifted gas stream;
- feeding at least a portion of the shifted gas stream to a CO₂ removal section (F) to provide a CO₂-rich gas stream and a first CO₂-depleted gas stream, optionally a second flash gas stream;
- and further feeding at least one of
   i) at least a portion of the first flash gas stream from the methanol synthesis section (C),
   ii) at least a portion of the off-gas stream from the distillation section (D), to the reformer section (A), and/or to the shift section (E) as additional carbon comprising feed(s).

All aspects relating to the system set out above are equally applicable to the process using said system.

In the process according to the invention, the process may further comprise feeding at least a portion of the second flash gas stream from the CO₂ removal section (F), to the reformer section (A) as an additional carbon comprising feed.

In particular, where the system further comprises a hydrogen recovery section (G), said process may further comprise: feeding the first CO₂-depleted gas stream to the hydrogen recovery section (G) and separating the first CO₂-depleted gas stream into at least a first hydrogen-rich stream, and at least a first off-gas stream. Also, the process may further comprise feeding at least a first portion of the first off-gas stream to the reformer section (A) as an additional carbon comprising feed. The process may also comprise providing at least a first portion of the hydrogen-rich stream to the methanol synthesis section (C), preferably in admixture with the second synthesis gas stream.

Additionally, in systems wherein the system further comprises a fuel system (H), at least a portion of the hydrogen-rich gas stream and/or the off-gas stream from the hydrogen recovery section (G) may be fed as fuel to the fuel system (H) and/or an auxiliary steam boiler and/or a gas turbine. Production of the first synthesis gas in the process of the invention may comprise an adiabatic prereforming step.

Additionally, or alternatively, for embodiments wherein the system further comprises a fuel system (H), at least a portion of the first CO₂-depleted gas stream can be split into at least a second CO₂-depleted gas stream and at least a third CO₂-depleted gas stream. In this embodiment, at least a portion of said second CO₂-depleted gas stream can be provided as a fuel feed to said fuel system (H).

In an alternative preferred embodiment, the principle of the invention can be used to achieve a smaller reduction of CO₂ emissions as for example in refurbishment of existing plants. A process for producing methanol in such systems may comprise splitting at least a portion of the first CO₂-depleted gas stream into at least a second CO₂-depleted gas stream and at least a third CO₂-depleted gas stream and optionally providing at least a portion of said second CO₂-depleted gas stream to the methanol synthesis section (C), preferably in admixture with the second synthesis gas stream.

The process may further comprise the step of: providing the third synthesis gas stream from said cooling train (B) at a higher temperature than the second synthesis gas stream preferably wherein the temperature of the third synthesis gas stream is at a temperature between 10°C greater than the dew point of the third synthesis gas stream, and 360°C.

The pressure of the third synthesis gas stream may be is increased by 2 to 6 bar prior to the inlet of the shift section (E). Furthermore, the steam feed may be mixed with the purge gas stream and/or the third synthesis gas stream and/or the first flash gas stream and/or the off-gas stream from said distillation section (D) prior to being inlet to the shift section (E).

Suitably, the molar steam/dry gas ratio at the inlet of the shift section (E) is in the range from 0.1 to 1.0, preferably 0.3 to 0.8. Also, the CO₂-depleted gas stream provided from the CO₂ removal section may have a CO₂ content below 1000 ppm vol., preferably below 500 ppm vol., more preferably below 20 ppm vol.. The CO₂-rich gas stream may comprise >90% vol. CO₂, such as > 98% vol. CO₂, preferably > 99% vol. CO₂.

The process suitably provides that the second synthesis gas stream is mixed with a hydrogen-rich stream in a ratio such that the provided admixed synthesis gas stream has a module between 1.8 and 2.2, preferably between 1.9 and 2.1. The carbon content in the combined flue gases from burning hydrogen-rich gas, off-gas and hydrocarbon fuel to the system is typically less than 3% of the sum of carbon content in the hydrocarbon feed and hydrocarbon fuel.

### Detailed description of drawings

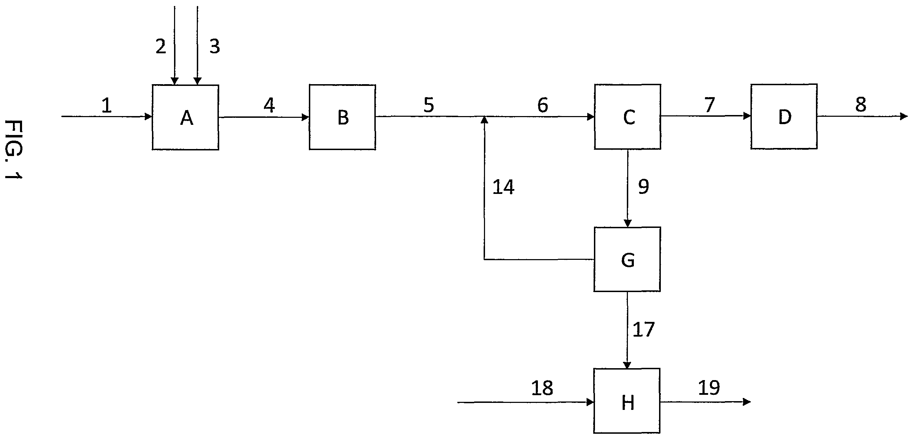

Figure 1 is a schematic illustration of a typical prior art system for methanol production.

In this embodiment, a hydrocarbon stream 1 is sent to a reformer section A. A steam feed 2 and/or an oxygen feed 3 is added to reformer section A as required by the selected reforming technology. The reformer section A may comprise one or more prereformer(s), steam reformer(s) and autothermal reformer(s) or any combinations thereof. The reformer section A provides a first synthesis gas stream 4.

The first synthesis gas stream 4 is sent to a cooling train B where the cooling train B provides sensible heat to produce steam, superheat steam, re-boiling duty for distillation and water preheat.

Most of the water in the gas in the cooling step B is condensed and separated before the second synthesis gas stream 5 is mixed with a hydrogen-rich stream 14 and sent to the methanol synthesis section C. The hydrogen-rich stream 14 is adjusted to ensure a module M above 1.8 in the mixed stream 6.

The methanol synthesis section C can be arranged to perform any methanol synthesis known in the art, so as to convert a portion of the second synthesis gas 5 or 6 to a raw methanol stream 7 and a purge gas 9. This conversion can for example be achieved by compressing the second synthesis gas and send it through a boiling water reactor where a portion of the CO, CO₂ and H₂ is converted to methanol followed by a condensation section separating the methanol in a liquid phase. A portion of the purge gas 9 can be recycled to the boiling water reactor (not shown). The raw methanol stream 7 is sent to a distillation section D where methanol of any desired quality in the product stream 8 can be made.

The purge gas 9 is sent to a hydrogen recovery section G. The hydrogen recovery section G converts the purge gas 9 to a hydrogen-rich stream 14 and an off-gas stream 17. The conversion is achieved either by using a PSA or a membrane. The hydrogen-rich stream 14 is used to adjust the module M of the second synthesis gas stream 6. The off-gas stream 17 is used as fuel in the system H.

If the module M in the second synthesis gas stream 5 is above 2 then addition of the hydrogen-rich stream 14 is not required. In this case the hydrogen recovery section G can be removed and the purge gas 9 be sent directly to a fuel system H (not illustrated).

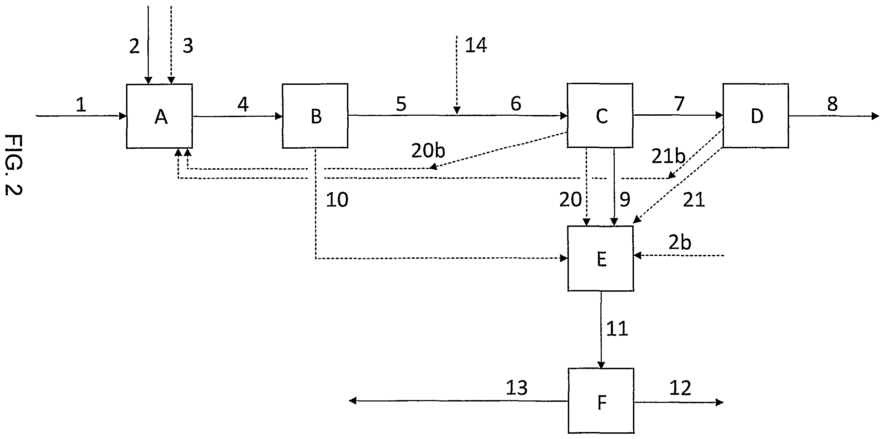

Figure 2 is a schematic illustration of a specific embodiment of this invention.

In this embodiment, a purified hydrocarbon stream 1 is mixed with a steam feed 2 such that the molar steam/carbon ratio is between 0.35 and 2.0, preferably between 0.5 and 1.5. Alternatively, a purified hydrocarbon feed is mixed with a steam feed and with at least one of i) at least a portion of the first flash gas stream from the methanol synthesis section (C), ii) at least a portion of the off-gas stream from the distillation section (D) to obtain a molar steam/carbon ratio between 0.35 and 2.0. The mixture of the hydrocarbon stream 1 and steam feed 2 optionally also comprising one or more of i) and/or ii) is fed to a reformer section A. The stream feeds 1 and 2 optionally also one or more of i) and/or ii) are sent through a prereforming reactor where hydrocarbons except for CH₄ are reduced to less than 0.5 mol %, preferably less than 0.2 mol%. The gas from the prereformer is optionally sent through a heated tubular reformer for further conversion of CH₄ to CO, CO₂ and H₂. The gas from the prereformer or - when present - the gas from the tubular reformer is sent through an autothermal reformer where also an oxygen feed 3 with a purity higher than 90 mol% preferably higher than 98 mol% is added. The reformer section A provides a first synthesis gas stream 4.

The first synthesis gas stream 4 is sent to a cooling train B where the cooling train B provides sensible heat to produce steam, superheat steam, re-boiling duty for distillation and water preheat. The cooling train B converts the first synthesis gas 4 to a second synthesis gas stream 5 and a third synthesis gas stream 10. The third synthesis gas stream 10 is sent to a shift section E.

The second synthesis gas stream 5, provided from the cooling train B, is optionally mixed with a hydrogen-rich stream 14 and sent to the methanol synthesis section C. The hydrogen-rich stream 14 is adjusted to ensure a module M above 1.8, preferably of 2.05, in the mixed stream 6. The hydrogen-rich stream can be provided by an external feed of hydrogen or by other streams isolated downstream the methanol synthesis section C (not shown). The mixed stream 6 is compressed to 70 - 100 bar g, preferably 80 to 90 bar g upstream to the methanol synthesis section C inlet.

The methanol synthesis section C can be arranged to perform any methanol synthesis known in the art, so as to convert part of the second synthesis gas 5 or 6 to a raw methanol stream 7 and a purge gas 9. This can for example be achieved by compressing the second synthesis gas 5 or 6 and sending the compressed second synthesis gas 5 or 6 through a boiling water reactor. In the boiling water reactor, at least a portion of the CO, CO₂ and H₂ is converted to methanol while the heat of reaction is removed by cooling with boiling water. The exit gas from the boiling water reactor is cooled to condense out at least a portion of the methanol and thereby make a liquid raw methanol stream 7.

The raw methanol stream 7 is sent to a distillation section D. The distillation section D can be arranged to provide methanol of any desired quality in the product stream 8. Said distillation further provides an off-gas stream 21 and/or 21b. The distillation can for example be achieved using a distillation column controlling the column temperature and pressure to exploit differences in relative volatility of the components. The off-gas stream from the distillation section can be arranged to be recycled such that a portion 21b of the off-gas is fed to the reformer section A as an additional carbon comprising feed and/or a portion 21 of the off-gas is fed to the shift section E as an additional carbon comprising feed.

At least a portion of the purge gas 9 is mixed with the third synthesis gas 10 and optionally with steam feed 2b, optionally with the first flash gas stream 20 from the methanol synthesis section C and/or optionally with the off-gas stream 21 form the distillation section D, optionally before the combined mixed gas is sent to the shift section E. The pressure of the third synthesis gas stream 10 is preferably increased by 2 to 6 bar before the shift reactor E inlet or before being mixed with other feeds. The steam addition is adjusted to give a molar steam/dry gas ratio of the combined stream between 0.1 to 1.0 preferably between 0.3 and 0.8. The combined stream is sent through a MT shift reactor with an inlet temperature between 200°C and 250°C, preferably 210°C. Alternatively the combined stream is sent through one or more MT shift reactors. In the MT shift reactor(s), CO and H₂O is converted to CO₂ and H₂ in accordance with the shift equilibrium reaction. The reaction takes place under adiabatic conditions. The conversion is therefore limited by the adiabatic equilibrium temperature. The shift section E provides a shifted gas stream 11. At least a portion of the shifted gas stream 11 is sent to a CO₂ removal section F, which provides a CO₂-rich gas stream 12, a first CO₂-depleted gas stream 13 and optionally a second flash gas stream 12b. The CO₂-depleted gas stream 13 has preferably a CO₂-content below 1%, preferably below 0.1%. Any CO₂ removal process known in the art can be used. The CO₂-rich gas stream 12 can be sent to storage and/or used for other purposes.

In this embodiment, multiple streams are optionally recycled to the reformer section A and/or the shift section E. However, at least one of i) at least a portion of the first flash gas stream (20, 20b) from the methanol synthesis section (C), ii) at least a portion of the off-gas stream (21, 21b) from the distillation section (D), is/are arranged to be fed as additional carbon comprising feed(s) to the reformer section (A) and/or to the shift section (E).

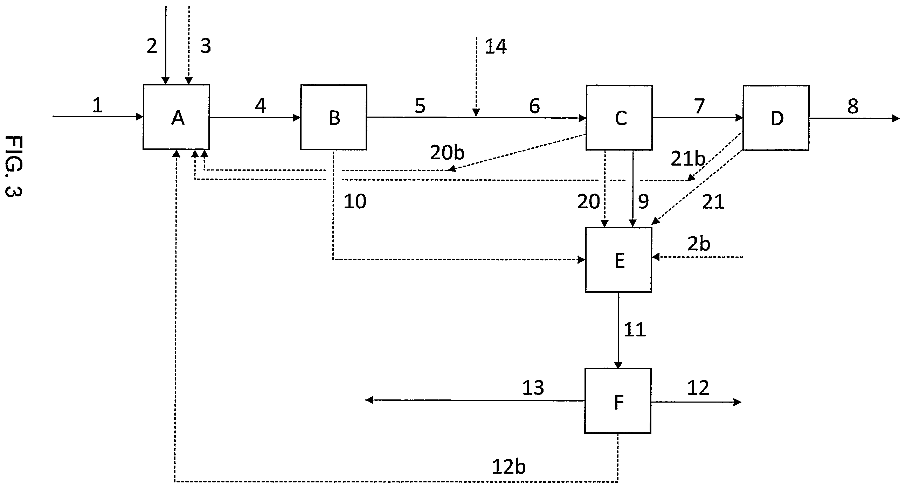

Figure 3 is a schematic illustration of a specific embodiment of this invention.

This embodiment comprises all elements from Figure 2 i.e., feeds and sections, however, in addition, this embodiment comprises that at least a portion of the second flash gas stream 12b from the CO₂ removal section F, is arranged to be fed to the reformer section A as an additional carbon comprising feed.

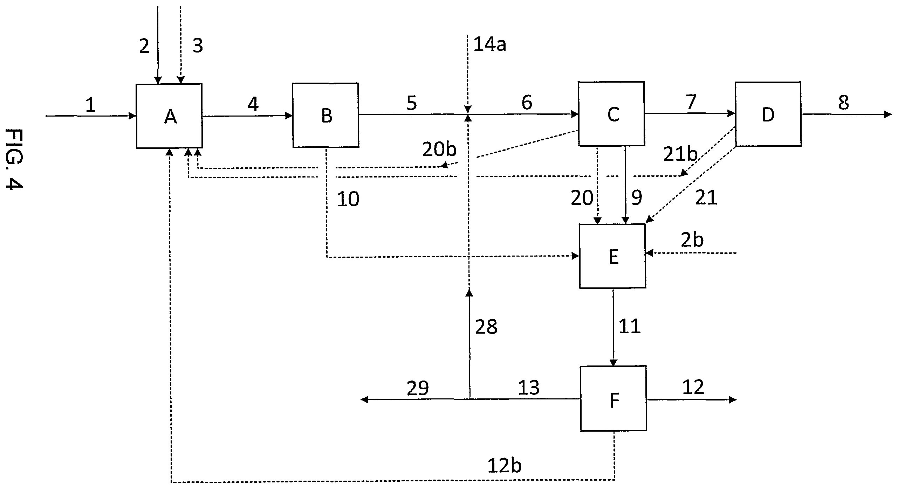

Figure 4 is a schematic illustration of a specific embodiment of this invention.

This embodiment comprises all elements from Figure 2 or 3 i.e., feeds and sections, however in this alternative embodiment the first CO₂-depleted gas stream 13 is split in at least a second 28 and a third 29 CO₂-depleted gas streams. The second CO₂-depleted gas stream 28 is arranged to be provided to the methanol synthesis section C, preferably in admixture 6 with the second synthesis gas stream 5. In this way, the second CO₂-depleted gas stream 28 serves as an H₂ source for the methanol synthesis such that the CO₂-depleted gas stream 28 is an alternative to a hydrogen-rich stream 14a provided from an external H₂ source.

The third 29 CO₂-depleted gas stream can be used as fuel.

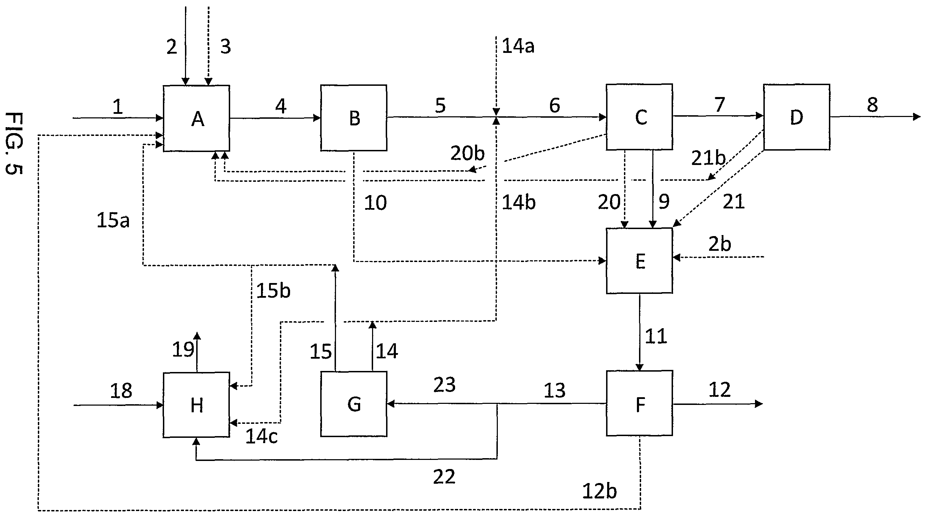

Figure 5 is a schematic illustration of a specific embodiment of this invention.

This embodiment comprises all elements from Figure 2 or 3 i.e., feeds and sections, however, in addition, this embodiment comprises a hydrogen recovery section G arranged to receive the CO₂-depleted gas stream 13 from the CO₂ removal section F and this embodiment comprises a fuel system H.

The hydrogen recovery section G converts at least a portion of the CO₂-depleted gas stream 13 to at least a first hydrogen-rich stream 14, and at least a first off-gas stream 15. This is preferably achieved using a pressure swing adsorption (PSA) unit or a membrane, one of which is comprised in the hydrogen recovery section G. The first off-gas stream 15 may sent as an alternative carbon comprising feed 15a to the reformer section A. The first hydrogen-rich stream 14 may be used to adjust the module M of the second synthesis gas stream 5 or 6, as stream 14b i.e. the first hydrogen-rich stream 14 may be sent to the methanol synthesis section C as hydrogen-rich stream 14b, preferably in an admixture 6 with the second synthesis gas stream 5 hereby providing an alternative to the H₂ source of an external hydrogen-rich stream 14a and to the second CO₂-depleted gas stream 28 provided from the system itself (embodiment illustrated in Figure 4). Depending on the selected technology in the hydrogen recovery section G, i.e., PSA or membrane, it will be required to compress the first hydrogen-rich stream 14b and/or the first off-gas stream 15 before feeding the first hydrogen-rich stream 14b to methanol synthesis section C and the first off-gas stream 15a to the reformer section A.

Alternatively, within this embodiment, the hydrogen recovery section G converts at least a portion of the CO₂-depleted gas stream 13 to a first hydrogen-rich stream 14, and a first off-gas stream 15 and the hydrogen recovery section G further provides a second portion 15b of the first off-gas stream 15 and/or a second portion 14c of the hydrogen-rich stream 14. The second portion 15b of the first off-gas stream 15 and/or the second portion 14c of the hydrogen-rich stream 14 may be sent as fuel feeds to the fuel system H.

Alternatively, or additionally, the first CO₂-depleted gas stream 13 from the CO₂ removal section F is split into at least a second CO₂-depleted gas stream 22 and a third CO₂-depleted gas stream 23, and a portion of the second CO₂-depleted gas stream 22 may be send as a fuel feed to the fuel system H.

An external hydrocarbon fuel 18 is used for operating the burner pilots and as back up fuel in the fuel system H. The fuel system H supplies the required fuel for elements of the plant, e.g., a tubular reformer, a fired heater, an auxiliary boiler or a gas turbine. The resulting flue gas stream 19 may be emitted to the atmosphere.

The flow of the third synthesis gas stream 10 is adjusted to ensure that the carbon content in the flue gas stream 19 is less than 3% of the combined carbon content in the hydrocarbon stream 1 and the hydrocarbon fuel 18.

### EXAMPLE 1

The data in table 1 illustrates the effect of the invention on three existing methanol synthesis production layouts.

In case 1 the methanol production was provided from a system layout without a shift section (E) and without a CO₂ removal section (F). In this way, case 1 provides a reference for the carbon emission emitted from a standard methanol production system.

In case 2 and 3 the methanol production was provided from a system layout, comprising a shift section (E) and a CO₂ removal section (F). In both cases, the first flash gas stream (20) from the methanol section (C) and the off-gas stream (21) from the distillation section (D) were arranged to be provided to the shift section (E). Additionally, in both cases a portion of the off-gas stream (15a) from the hydrogen recovery section (G) was fed as a carbon comprising feed to reformer section (A). More specifically, in case 2, a portion of the off-gas stream (15a) from the hydrogen recovery section (G) was fed as a carbon comprising feed to reformer (A) and a portion of the off-gas stream (15b) was fed to the fuel section (H). In case 3 substantially all of the off-gas (15) from the hydrogen recovery section (G) was fed as a carbon comprising feed to the reformer section (A).

The capacity of the methanol plants is in all cases 5000 MTPD methanol (given as 100 % methanol).

The carbon emissions % is the carbon content emitted from the system i.e., carbon content in the flue gas stream relative to the total carbon content of the incoming feeds. Hence, the result presented as carbon emission % is the carbon in stream 19 divided by carbon in stream 1+18, times 100%.

**Table 1**

| Case | Carbon in stream 1+18 kNm3/h | Carbon in stream 19 kNm3/h | Carbon in stream 12 kNm3/h | Carbon emission % |
|---|---|---|---|---|
| 1 | 174.3 | 28.8 | 0.0 | 16.5 |
| 2 | 181.1 | 4.8 | 30.6 | 2.6 |
| 3 | 183.4 | 0.9 | 36.8 | 0.5 |

The results in Table 1 show that the invention has a significant impact on the CO₂ emission. The carbon emission in cases 2 and 3 show significant improvement, such as achieving a carbon emission of less than 3 % of the total carbon content of the incoming fees. In a preferred embodiment, exemplified by case 3, the carbon emission is even less than 1%.

## Claims

1. A system (100) for producing methanol, said system (100) comprising:
- a hydrocarbon feed (1);
- a steam feed (2);
- optionally, an oxygen feed (3);
- a reformer section (A) arranged to receive said hydrocarbon feed (1), said steam feed (2) and, where present, said oxygen feed (3), and to provide a first synthesis gas stream (4);
- a cooling train (B), arranged to receive at least a portion of the first synthesis gas stream (4), and provide a second synthesis gas stream (5) and a third synthesis gas stream (10);
- a methanol synthesis section (C) arranged to receive at least a portion of the second synthesis gas stream (5), optionally in admixture (6) with a hydrogen-rich stream (14), and to provide a raw methanol stream (7), a first flash gas stream (20) and a purge gas stream (9);
- a distillation section (D) arranged to receive at least a portion of the raw methanol stream (7) and to provide a product methanol stream (8) and an off-gas stream (21);
- a shift section (E) arranged to receive at least a portion of said purge gas stream (9), at least a portion of said third synthesis gas stream (10), optionally a steam feed (2b), and provide a shifted gas stream (11);
- a CO₂ removal section (F) arranged to receive at least a portion of the shifted gas stream (11) and provide a CO₂-rich gas stream (12) and a first CO₂-depleted gas stream (13), optionally a second flash gas stream (12b);
wherein at least one of
i) at least a portion of the first flash gas stream (20, 20b) from the methanol synthesis section (C),
ii) at least a portion of the off-gas stream (21, 21b) from the distillation section (D),
is/are arranged to be fed as additional carbon comprising feed(s) to the reformer section (A), and/or to the shift section (E).

2. The system according to claim 1, wherein at least a portion of the second flash gas stream (12b) from the CO₂ removal section (F), is arranged to be fed to the reformer section (A) as an additional carbon comprising feed.

3. The system according to any one of the preceding claims, wherein the shift section (E) comprises one or more shift reactors.

4. The system according to any one of the preceding claims, further comprising a hydrogen recovery section (G), said hydrogen recovery section (G) being arranged to receive at least a portion of the first CO₂-depleted gas stream (13), and to provide at least a first hydrogen-rich stream (14), and at least a first off-gas stream (15).

5. The system according to claim 4, wherein said system is arranged to provide at least a first portion (15a) of the first off-gas stream (15) to the reformer section (A) as an additional carbon comprising feed.

6. The system according to any one of claims 4 or 5, wherein the system is arranged to provide at least a first portion (14b) of the hydrogen-rich stream (14) to the methanol synthesis section (C), preferably in admixture (6) with the second synthesis gas stream (5).

7. The system according to any one of claims 4 to 6, wherein the system further comprises a fuel system (H) and wherein said hydrogen recovery section (G) is arranged to provide a second portion (15b) of the first off-gas stream (15) and/or a second portion (14c) of the hydrogen-rich stream (14), wherein at least a portion of said second portion (15b) of the first off-gas stream (15) and/or at least a portion of said second portion (14c) of the hydrogen-rich stream (14) is arranged to be fed as a fuel feed to said fuel system (H) and/or an auxiliary steam boiler and/or a gas turbine.

8. The system according to any one of the preceding claims, wherein the system further comprises a fuel system (H) and wherein at least a portion of the first CO₂-depleted gas stream (13) is arranged to be split into at least a second CO₂-depleted gas stream (22) and at least a third CO₂-depleted gas stream (23), wherein at least a portion of said second CO₂-depleted gas stream (22) is arranged to be fed as a fuel feed to said fuel system (H).

9. The system according to any one of claims 1 to 3, wherein at least a portion of the first CO₂-depleted gas stream (13) is arranged to be split into at least a second CO₂-depleted gas stream (28) and at least a third CO₂-depleted gas stream (29) wherein at least a portion of said second CO₂-depleted gas stream (28) is arranged to be provided to the methanol synthesis section (C), preferably in admixture (6) with the second synthesis gas stream (5).

10. The system according to any one of the preceding claims, wherein the cooling train (B) is arranged to provide sensible heat to produce steam, superheat steam, re-boiling duty for distillation and water preheat and/or allow for separation of condensed H₂O.

11. The system according to any one of the preceding claims, wherein cooling train (B) is arranged to provide the third synthesis gas stream (10) at a higher temperature than the second synthesis gas stream (5, 6), preferably wherein the temperature of the third synthesis gas stream (10) is between 10°C greater than the dew point of the third synthesis gas stream (10), and 360°C.

12. The system according to any one of the preceding claims, wherein the second synthesis gas stream (5, 6) has a module between 1.8 and 2.2, preferably between 1.9 and 2.1.

13. The system according to any one of the preceding claims, wherein said steam feed (2b) is arranged to be mixed with the purge gas stream (9) and/or the third synthesis gas stream (10) and/or the first flash gas stream (20) and/or the off-gas stream (21) from said distillation section (D) prior to being inlet to the shift section (E).

14. A process for producing methanol in a system (100) according to any one of the preceding claims, said process comprising the steps of:
- feeding the hydrocarbon feed (1), the steam feed (2) and, where present, the oxygen feed (3) to the reformer section (A) to provide a first synthesis gas stream (4);
- feeding at least a portion of the first synthesis gas stream (4) to the cooling train (B) to provide a second synthesis gas (5) and a third synthesis gas (10);
- feeding at least a portion of the second synthesis gas stream (5), optionally in admixture (6) with a hydrogen-rich stream (14), to a methanol synthesis section (C) and converting at least a portion of the second synthesis gas stream (5, 6) to a raw methanol stream (7), a first flash gas stream (20) and a purge gas stream (9);
- feeding at least a portion of the raw methanol stream (7) to a distillation section (D) to provide a product methanol stream (8) and an off-gas stream (21);
- feeding at least a portion of the purge gas stream (9), at least a portion of the third synthesis gas stream (10), optionally a steam feed (2b), to the shift section (E), to provide a shifted gas stream (11);
- feeding at least a portion of the shifted gas stream (11) to a CO₂ removal section (F) to provide a CO₂-rich gas stream (12) and a first CO₂-depleted gas stream (13), optionally a second flash gas stream (12b);
- and further feeding at least one of
i) at least a portion of the first flash gas stream (20, 20b) from the methanol synthesis section (C),
ii) at least a portion of the off-gas stream (21, 21b) from the distillation section (D),
to the reformer section (A), and/or to the shift section (E) as additional carbon comprising feed(s).

15. The process according to claim 14, wherein said process further comprises feeding at least a portion of the second flash gas stream (12b) from the CO₂ removal section (F), to the reformer section (A) as an additional carbon comprising feed.

16. The process according to any one of claims 14 or 15, wherein the system further comprises a hydrogen recovery section (G), said process further comprising: feeding the first CO₂-depleted gas stream (13) to the hydrogen recovery section (G) and separating the first CO₂-depleted gas stream (13) into at least a first hydrogen-rich stream (14), and at least a first off-gas stream (15).

17. The process according to claim 16, wherein said process further comprising: feeding at least a first portion (15a) of the first off-gas stream (15) to the reformer section (A) as an additional carbon comprising feed.

18. The process according to any one of claim 16 or 17, wherein said process comprises providing at least a first portion (14b) of the hydrogen-rich stream (14) to the methanol synthesis section (C), preferably in admixture (6) with the second synthesis gas stream (5).

19. The process according to any one of claims 16 to 18, wherein the system further comprises a fuel system (H) and wherein a second portion (15b) of the first off-gas stream (15) and/or a second portion (14c) of the hydrogen-rich stream (14), are provided as fuel to the fuel system (H) and/or an auxiliary steam boiler and/or a gas turbine.

20. The process according to any one of claims 14-19, wherein the system further comprises a fuel system (H), and wherein at least a portion of the first CO₂-depleted gas stream (13) is split into at least a second CO₂-depleted gas stream (22) and at least a third CO₂-depleted gas stream (23) and wherein at least a portion of said second CO₂-depleted gas stream (22) is provided as a fuel feed to said fuel system (H).

21. The process according to any one of claims 14-15, wherein at least a portion of the first CO₂-depleted gas stream (13) is split into at least a second CO₂-depleted gas stream (28) and at least a third CO₂-depleted gas stream (29) and wherein at least a portion of said second CO₂-depleted gas stream (28) is provided to the methanol synthesis section (C), preferably in admixture (6) with the second synthesis gas stream (5).

22. The process according to any one of claims 14 to 21, comprising the step of: providing the third synthesis gas stream (10) from said cooling train (B) at a higher temperature than the second synthesis gas stream (5, 6), preferably wherein the temperature of the third synthesis gas stream (10) is between 10°C greater than the dew point of the third synthesis gas stream (10), and 360°C.

23. The process according to any one of claims 14 to 22, wherein said steam feed (2b) is mixed with the purge gas stream (9) and/or the third synthesis gas stream (10), and/or the first flash gas stream (20) and/or the off-gas stream (21) from said distillation section (D) prior to being inlet to the shift section (E).

24. The process according to any one of claims 14 to 23, wherein the carbon content in the combined flue gases (19) from burning a portion of said second portion (15b) of the first off-gas stream (15) and/or a portion of a second portion (14c) of the hydrogen-rich stream (14) and hydrocarbon fuel (18) to the system is less than 3% of the sum of carbon content in the hydrocarbon feed (1) and hydrocarbon fuel (18).

25. A plant comprising the system according to any one of claims 1 to 13.

## Patentansprüche

1. System (100) zum Herstellen von Methanol, wobei das System (100) umfasst:
- eine Kohlenwasserstoffzufuhr (1);
- eine Dampfzufuhr (2);
- wahlweise eine Sauerstoffzufuhr (3);
- eine Reformersektion (A), die dafür angeordnet ist, die Kohlenwasserstoffzufuhr (1), die Dampfzufuhr (2) und, wo vorhanden, die Sauerstoffzufuhr (3) aufzunehmen und einen ersten Synthesegasstrom (4) bereitzustellen;
- einen Kühlstrang (B), der dafür angeordnet ist, mindestens einen Teil des ersten Synthesegasstroms (4) aufzunehmen und einen zweiten Synthesegasstrom (5) und einen dritten Synthesegasstrom (10) bereitzustellen;
- eine Methanolsynthesesektion (C), die dafür angeordnet ist, mindestens einen Teil des zweiten Synthesegasstroms (5), wahlweise in Gemisch (6) mit einem wasserstoffreichen Strom (14), aufzunehmen und einen Rohmethanolstrom (7), einen ersten Flash-Gasstrom (20) und einen Spülgasstrom (9) bereitzustellen;
- eine Destillationssektion (D), die dafür angeordnet ist, mindestens einen Teil des Rohmethanolstroms (7) aufzunehmen und einen Produktmethanolstrom (8) und einen Abgasstrom (21) bereitzustellen;
- eine Konvertierungssektion (E), die dafür angeordnet ist, mindestens einen Teil des Spülgasstroms (9), mindestens einen Teil des dritten Synthesegasstroms (10), wahlweise eine Dampfzufuhr (2b), aufzunehmen und einen konvertierten Gasstrom (11) bereitzustellen;
- eine CO₂-Entfernungssektion (F), die dafür angeordnet ist, mindestens einen Teil des konvertierten Gasstroms (11) aufzunehmen und einen CO₂-reichen Gasstrom (12) und einen ersten CO₂-verarmten Gasstrom (13), wahlweise einen zweiten Flash-Gasstrom (12b), bereitzustellen;
wobei mindestens einer von
i) mindestens einem Teil des ersten Flash-Gasstroms (20, 20b) aus der Methanolsynthesesektion (C),
ii) mindestens einem Teil des Abgasstroms (21, 21b) aus der Destillationssektion (D),
dafür angeordnet ist/sind, als zusätzliche kohlenstoffhaltige Zufuhr(en) der Reformersektion (A) und/oder der Konvertierungssektion (E) zugeführt zu werden.

2. System nach Anspruch 1, wobei mindestens ein Teil des zweiten Flash-Gasstroms (12b) aus der CO₂-Entfernungssektion (F) dafür angeordnet ist, der Reformersektion (A) als eine zusätzliche kohlenstoffhaltige Zufuhr zugeführt zu werden.

3. System nach einem der vorstehenden Ansprüche, wobei die Konvertierungssektion (E) einen oder mehrere Konvertierungsreaktoren umfasst.

4. System nach einem der vorstehenden Ansprüche, weiter umfassend eine Wasserstoffrückgewinnungssektion (G), wobei die Wasserstoffrückgewinnungssektion (G) dafür angeordnet ist, mindestens einen Teil des ersten CO₂-verarmten Gasstroms (13) aufzunehmen und mindestens einen ersten wasserstoffreichen Strom (14) und mindestens einen ersten Abgasstrom (15) bereitzustellen.

5. System nach Anspruch 4, wobei das System dafür angeordnet ist, der Reformersektion (A) mindestens einen ersten Teil (15a) des ersten Abgasstroms (15) als zusätzliche kohlenstoffhaltige Zufuhr zuzuführen.

6. System nach einem der Ansprüche 4 oder 5, wobei das System dafür angeordnet ist, mindestens einen ersten Teil (14b) des wasserstoffreichen Stroms (14) der Methanolsynthesesektion (C) zuzuführen, vorzugsweise in Gemisch (6) mit dem zweiten Synthesegasstrom (5).

7. System nach einem der Ansprüche 4 bis 6, wobei das System weiter ein Brennstoffsystem (H) umfasst und wobei die Wasserstoffrückgewinnungssektion (G) dafür angeordnet ist, einen zweiten Teil (15b) des ersten Abgasstroms (15) und/oder einen zweiten Teil (14c) des wasserstoffreichen Stroms (14) bereitzustellen, wobei mindestens ein Teil des zweiten Teils (15b) des ersten Abgasstroms (15) und/oder mindestens ein Teil des zweiten Teils (14c) des wasserstoffreichen Stroms (14) dafür angeordnet ist, dem Brennstoffsystem (H) als Brennstoff und/oder einem Hilfsdampfkessel und/oder einer Gasturbine zugeführt zu werden.

8. System nach einem der vorstehenden Ansprüche, wobei das System weiter ein Brennstoffsystem (H) umfasst und wobei mindestens ein Teil des ersten CO₂-verarmten Gasstroms (13) dafür angeordnet ist, in mindestens einen zweiten CO₂-verarmten Gasstrom (22) und mindestens einen dritten CO₂-verarmten Gasstrom (23) aufgeteilt zu werden, wobei mindestens ein Teil des zweiten CO₂-verarmten Gasstroms (22) dafür angeordnet ist, dem Brennstoffsystem (H) als eine Brennstoffzufuhr zugeführt zu werden.

9. System nach einem der Ansprüche 1 bis 3, wobei mindestens ein Teil des ersten CO₂-verarmten Gasstroms (13) dafür angeordnet ist, in mindestens einen zweiten CO₂-verarmten Gasstrom (28) und mindestens einen dritten CO₂-verarmten Gasstrom (29) aufgeteilt zu werden, wobei mindestens ein Teil des zweiten CO₂-verarmten Gasstroms (28) dafür angeordnet ist, der Methanolsynthesesektion (C) zugeführt zu werden, vorzugsweise in Gemisch (6) mit dem zweiten Synthesegasstrom (5).

10. System nach einem der vorstehenden Ansprüche, wobei der Kühlstrang (B) dafür angeordnet ist, fühlbare Wärme zum Erzeugen von Dampf, überhitztem Dampf, Wiederverdampfungsleistung für die Destillation und Wasservorwärmung bereitzustellen und/oder die Abtrennung von kondensiertem H₂O zu ermöglichen.

11. System nach einem der vorstehenden Ansprüche, wobei der Kühlstrang (B) dafür angeordnet ist, den dritten Synthesegasstrom (10) bei einer höheren Temperatur als den zweiten Synthesegasstrom (5, 6) bereitzustellen, vorzugsweise, wobei die Temperatur des dritten Synthesegasstroms (10) zwischen 10 °C über dem Taupunkt des dritten Synthesegasstroms (10) und 360 °C liegt.

12. System nach einem der vorstehenden Ansprüche, wobei der zweite Synthesegasstrom (5, 6) einen Modul zwischen 1,8 und 2,2, vorzugsweise zwischen 1,9 und 2,1, aufweist.

13. System nach einem der vorstehenden Ansprüche, wobei die Dampfzufuhr (2b) dafür angeordnet ist, mit dem Spülgasstrom (9) und/oder dem dritten Synthesegasstrom (10) und/oder dem ersten Flash-Gasstrom (20) und/oder dem Abgasstrom (21) aus der Destillationssektion (D) vermischt zu werden, bevor er in die Konvertierungssektion (E) eingelassen wird.

14. Prozess zum Herstellen von Methanol in einem System (100) nach einem der vorstehenden Ansprüche, wobei der Prozess die folgenden Schritte umfasst:
- Zuführen der Kohlenwasserstoffzufuhr (1), der Dampfzufuhr (2) und, wo vorhanden, der Sauerstoffzufuhr (3) zur Reformersektion (A), um einen ersten Synthesegasstrom (4) bereitzustellen;
- Zuführen mindestens eines Teils des ersten Synthesegasstroms (4) zum Kühlstrang (B), um ein zweites Synthesegas (5) und ein drittes Synthesegas (10) bereitzustellen;
- Zuführen mindestens eines Teils des zweiten Synthesegasstroms (5), wahlweise in Gemisch (6) mit einem wasserstoffreichen Strom (14), zu einer Methanolsynthesesektion (C) und Umwandeln mindestens eines Teils des zweiten Synthesegasstroms (5, 6) in einen Rohmethanolstrom (7), einen ersten Flash-Gasstrom (20) und einen Spülgasstrom (9);
- Zuführen mindestens eines Teils des Rohmethanolstroms (7) zu einer Destillationssektion (D), um einen Produktmethanolstrom (8) und einen Abgasstrom (21) bereitzustellen;
- Zuführen mindestens eines Teils des Spülgasstroms (9), mindestens eines Teils des dritten Synthesegasstroms (10), wahlweise einer Dampfzufuhr (2b), zur Konvertierungssektion (E), um einen konvertierten Gasstrom (11) bereitzustellen;
- Zuführen mindestens eines Teils des konvertierten Gasstroms (11) zu einer CO₂-Entfernungssektion (F), um einen CO₂-reichen Gasstrom (12) und einen ersten CO₂-verarmten Gasstrom (13), wahlweise einen zweiten Flash-Gasstrom (12b), bereitzustellen;
- und weiteres Zuführen mindestens eines von
i) mindestens einem Teil des ersten Flash-Gasstroms (20, 20b) aus der Methanolsynthesesektion (C),
ii) mindestens einem Teil des Abgasstroms (21, 21b) aus der Destillationssektion (D),
zur Reformersektion (A) und/oder zur Konvertierungssektion (E) als zusätzliche kohlenstoffhaltige Zufuhr(en).

15. Prozess nach Anspruch 14, wobei der Prozess weiter das Zuführen mindestens eines Teils des zweiten Flash-Gasstroms (12b) aus der CO₂-Entfernungssektion (F) zur Reformersektion (A) als eine zusätzliche kohlenstoffhaltige Zufuhr umfasst.

16. Prozess nach einem der Ansprüche 14 oder 15, wobei das System weiter eine Wasserstoffrückgewinnungssektion (G) umfasst, wobei der Prozess weiter umfasst: Zuführen des ersten CO₂-verarmten Gasstroms (13) zur Wasserstoffrückgewinnungssektion (G) und Trennen des ersten CO₂-verarmten Gasstroms (13) in mindestens einen ersten wasserstoffreichen Strom (14) und mindestens einen ersten Abgasstrom (15).

17. Prozess nach Anspruch 16, wobei der Prozess weiter umfasst: Zuführen mindestens eines ersten Teils (15a) des ersten Abgasstroms (15) zu der Reformersektion (A) als eine zusätzliche kohlenstoffhaltige Zufuhr.

18. Prozess nach einem der Ansprüche 16 oder 17, wobei der Prozess das Zuführen mindestens eines ersten Teils (14b) des wasserstoffreichen Stroms (14) zur Methanolsynthesesektion (C) umfasst, vorzugsweise in Gemisch (6) mit dem zweiten Synthesegasstrom (5).

19. Prozess nach einem der Ansprüche 16 bis 18, wobei das System weiter ein Brennstoffsystem (H) umfasst und wobei ein zweiter Teil (15b) des ersten Abgasstroms (15) und/oder ein zweiter Teil (14c) des wasserstoffreichen Stroms (14) als Brennstoff dem Brennstoffsystem (H) und/oder einem Hilfsdampfkessel und/oder einer Gasturbine bereitgestellt werden.

20. Prozess nach einem der Ansprüche 14-19, wobei das System weiter ein Brennstoffsystem (H) umfasst und wobei mindestens ein Teil des ersten CO₂-verarmten Gasstroms (13) in mindestens einen zweiten CO₂-verarmten Gasstrom (22) und mindestens einen dritten CO₂-verarmten Gasstrom (23) aufgeteilt wird und wobei mindestens ein Teil des zweiten CO₂-verarmten Gasstroms (22) dem Brennstoffsystem (H) als eine Brennstoffzufuhr bereitgestellt wird.

21. Prozess nach einem der Ansprüche 14-15, wobei mindestens ein Teil des ersten CO₂- verarmten Gasstroms (13) in mindestens einen zweiten CO₂-verarmten Gasstrom (28) und mindestens einen dritten CO₂-verarmten Gasstrom (29) aufgeteilt wird und wobei mindestens ein Teil des zweiten CO₂-verarmten Gasstroms (28) der Methanolsynthesesektion (C) bereitgestellt wird, vorzugsweise in Gemisch (6) mit dem zweiten Synthesegasstrom (5).

22. Prozess nach einem der Ansprüche 14 bis 21, umfassend den Schritt von: Bereitstellen des dritten Synthesegasstroms (10) aus dem Kühlstrang (B) mit einer höheren Temperatur als der zweite Synthesegasstrom (5, 6), vorzugsweise wobei die Temperatur des dritten Synthesegasstroms (10) zwischen 10 °C über dem Taupunkt des dritten Synthesegasstroms (10) und 360 °C liegt.

23. Prozess nach einem der Ansprüche 14 bis 22, wobei der Dampfstrom (2b) mit dem Spülgasstrom (9) und/oder dem dritten Synthesegasstrom (10), und/oder dem ersten Flash-Gasstrom (20) und/oder dem Abgasstrom (21) aus der Destillationssektion (D) vermischt wird, bevor er in die Konvertierungssektion (E) eingelassen wird.

24. Prozess nach einem der Ansprüche 14 bis 23, wobei der Kohlenstoffgehalt in den kombinierten Rauschgasen (19) vom Verbrennen eines Teils des zweiten Teils (15b) des ersten Abgasstroms (15) und/oder eines Teils eines zweiten Teils (14c) des wasserstoffreichen Stroms (14) und des Kohlenwasserstoffbrennstoffs (18) zu dem System kleiner ist als 3 % der Summe des Kohlenstoffgehalts in der Kohlenwasserstoffzufuhr (1) und dem Kohlenwasserstoffbrennstoff (18).

25. Anlage, die das System nach einem der Ansprüche 1 bis 13 umfasst.

## Revendications

1. Système (100) de production de méthanol, ledit système (100) comprenant :
- une alimentation en hydrocarbures (1) ;
- une alimentation en vapeur (2) ;
- éventuellement, une alimentation en oxygène (3) ;
- une section de reformage (A) conçue pour recevoir ladite alimentation en hydrocarbures (1), ladite alimentation en vapeur (2) et, le cas échéant, ladite alimentation en oxygène (3), et pour fournir un premier flux de gaz de synthèse (4) ;
- un train de refroidissement (B), conçu pour recevoir au moins une partie du premier flux de gaz de synthèse (4), et fournir un deuxième flux de gaz de synthèse (5) et un troisième flux de gaz de synthèse (10) ;
- une section de synthèse de méthanol (C) conçue pour recevoir au moins une partie du deuxième flux de gaz de synthèse (5), éventuellement en mélange (6) avec un flux riche en hydrogène (14), et pour fournir un flux de méthanol brut (7), un premier flux de gaz flash (20) et un flux de gaz de purge (9) ;
- une section de distillation (D) conçue pour recevoir au moins une partie du flux de méthanol brut (7) et pour fournir un flux de méthanol produit (8) et un flux de gaz résiduaire (21) ;
- une section de conversion (E) conçue pour recevoir au moins une partie dudit flux de gaz de purge (9), au moins une partie dudit troisième flux de gaz de synthèse (10), éventuellement une alimentation en vapeur (2b), et fournir un flux de gaz converti (11) ;
- une section d'élimination du CO₂ (F) conçue pour recevoir au moins une partie du flux de gaz converti (11) et fournir un flux de gaz enrichi en CO₂ (12) et un premier flux de gaz appauvri en CO₂ (13), éventuellement un deuxième flux de gaz flash (12b) ;
dans lequel au moins une parmi :
i) au moins une partie du premier flux de gaz flash (20, 20b) provenant de la section de synthèse de méthanol (C),
ii) au moins une partie du flux de gaz résiduaire (21, 21b) provenant de la section de distillation (D),
est conçue pour être introduite à titre d'alimentation supplémentaire comprenant du carbone dans la section de reformage (A), et/ou dans la section de conversion (E).

2. Système selon la revendication 1, dans lequel au moins une partie du deuxième flux de gaz flash (12b) provenant de la section d'élimination du CO₂ (F), est conçue pour être introduite dans la section de reformage (A) à titre d'alimentation supplémentaire comprenant du carbone.

3. Système selon l'une quelconque des revendications précédentes, dans lequel la section de conversion (E) comprend un ou plusieurs réacteurs de conversion.

4. Système selon l'une quelconque des revendications précédentes, comprenant en outre une section de récupération d'hydrogène (G), ladite section de récupération d'hydrogène (G) étant conçue pour recevoir au moins une partie du premier flux de gaz appauvri en CO₂ (13), et pour fournir au moins un premier flux riche en hydrogène (14), et au moins un premier flux de gaz résiduaire (15).

5. Système selon la revendication 4, dans lequel ledit système est conçu pour fournir au moins une première partie (15a) du premier flux de gaz résiduaire (15) à la section de reformage (A) à titre d'alimentation supplémentaire comprenant du carbone.

6. Système selon l'une quelconque des revendications 4 ou 5, dans lequel le système est conçu pour fournir au moins une première partie (14b) du flux riche en hydrogène (14) à la section de synthèse de méthanol (C), de préférence en mélange (6) avec le deuxième flux de gaz de synthèse (5).

7. Système selon l'une quelconque des revendications 4 à 6, dans lequel le système comprend en outre un système d'alimentation en combustible (H) et dans lequel ladite section de récupération d'hydrogène (G) est conçue pour fournir une seconde partie (15b) du premier flux de gaz résiduaire (15) et/ou une seconde partie (14c) du flux riche en hydrogène (14), dans lequel au moins une partie de ladite seconde partie (15b) du premier flux de gaz résiduaire (15) et/ou au moins une partie de ladite deuxième partie (14c) du flux riche en hydrogène (14) est conçue pour être introduite à titre d'alimentation en combustible dans ledit système de combustible (H) et/ou une chaudière à vapeur auxiliaire et/ou une turbine à gaz.

8. Système selon l'une quelconque des revendications précédentes, dans lequel le système comprend en outre un système d'alimentation en combustible (H) et dans lequel au moins une partie du premier flux de gaz appauvri en CO₂ (13) est conçue pour être séparée en au moins un deuxième flux de gaz appauvri en CO₂ (22) et au moins un troisième flux de gaz appauvri en CO₂ (23), dans lequel au moins une partie dudit deuxième flux de gaz appauvri en CO₂ (22) est conçue pour être introduite à titre d'alimentation en combustible dans ledit système d'alimentation en combustible (H).

9. Système selon l'une quelconque des revendications 1 à 3, dans lequel au moins une partie du premier flux de gaz appauvri en CO₂ (13) est conçue pour être séparée en au moins un deuxième flux de gaz appauvri en CO₂ (28) et au moins un troisième flux de gaz appauvri en CO₂ (29), dans lequel au moins une partie dudit deuxième flux de gaz appauvri en CO₂ (28) est conçue pour être fournie à la section de synthèse de méthanol (C), de préférence en mélange (6) avec le deuxième flux de gaz de synthèse (5).

10. Système selon l'une quelconque des revendications précédentes, dans lequel le train de refroidissement (B) est conçu pour fournir de la chaleur sensible afin de produire de la vapeur, de la vapeur surchauffée, un cycle de rebouillage pour la distillation et le préchauffage de l'eau et/ou permettre la séparation d'eau H₂O condensée.

11. Système selon l'une quelconque des revendications précédentes, dans lequel le train de refroidissement (B) est conçu pour fournir le troisième flux de gaz de synthèse (10) à une température plus élevée que le deuxième flux de gaz de synthèse (5, 6), de préférence dans lequel la température du troisième flux de gaz de synthèse (10) est comprise entre 10 °C au-dessus du point de rosée du troisième flux de gaz de synthèse (10) et 360 °C.

12. Système selon l'une quelconque des revendications précédentes, dans lequel le deuxième flux de gaz de synthèse (5, 6) présente un module compris entre 1,8 et 2,2, de préférence entre 1,9 et 2,1.

13. Système selon l'une quelconque des revendications précédentes, dans lequel ladite alimentation en vapeur (2b) est conçue pour être mélangée avec le flux de gaz de purge (9) et/ou le troisième flux de gaz de synthèse (10) et/ou le premier flux de gaz flash (20) et/ou le flux de gaz résiduaire (21) de ladite section de distillation (D) avant d'être introduit dans la section de conversion (E).

14. Procédé de production de méthanol dans un système (100) selon l'une quelconque des revendications précédentes, ledit procédé comprenant les étapes de :
- introduction de l'alimentation en hydrocarbures (1), de l'alimentation en vapeur (2) et, le cas échéant, de l'alimentation en oxygène (3) dans la section de reformage (A) pour fournir un premier flux de gaz de synthèse (4) ;
- introduction d'au moins une partie du premier flux de gaz de synthèse (4) dans le train de refroidissement (B) pour fournir un deuxième gaz de synthèse (5) et un troisième gaz de synthèse (10) ;
- introduction d'au moins une partie du deuxième flux de gaz de synthèse (5), éventuellement en mélange (6) avec un flux riche en hydrogène (14), dans une section de synthèse de méthanol (C) et conversion d'au moins une partie du deuxième flux de gaz de synthèse (5, 6) en un flux de méthanol brut (7), un premier flux de gaz flash (20) et un flux de gaz de purge (9) ;
- introduction d'au moins une partie du flux de méthanol brut (7) dans une section de distillation (D) pour fournir un flux de méthanol produit (8) et un flux de gaz résiduaire (21) ;
- introduction d'au moins une partie du flux de gaz de purge (9), d'au moins une partie du troisième flux de gaz de synthèse (10), éventuellement d'une alimentation en vapeur (2b), dans la section de conversion (E), pour fournir un flux de gaz converti (11) ;
- introduction d'au moins une partie du flux de gaz converti (11) dans une section d'élimination du CO₂ (F) pour fournir un flux de gaz enrichi en CO₂ (12) et un premier flux de gaz appauvri en CO₂ (13), éventuellement un deuxième flux de gaz flash (12b) ;
- et introduction ultérieure d'au moins une parmi
i) au moins une partie du premier flux de gaz flash (20, 20b) provenant de la section de synthèse de méthanol (C),
ii) au moins une partie du flux de gaz résiduaire (21, 21b) provenant de la section de distillation (D),
dans la section de reformage (A), et/ou dans la section de conversion (E) à titre d'alimentation supplémentaire comprenant du carbone.

15. Procédé selon la revendication 14, dans lequel ledit procédé consiste en outre à introduire au moins une partie du deuxième flux de gaz flash (12b) provenant de la section d'élimination du CO₂ (F), dans la section de reformage (A) à titre d'alimentation supplémentaire comprenant du carbone.

16. Procédé selon l'une quelconque des revendications 14 ou 15, dans lequel le système comprend en outre une section de récupération d'hydrogène (G), ledit procédé comprenant en outre : l'introduction du premier flux de gaz appauvri en CO₂ (13) dans la section de récupération d'hydrogène (G) et la séparation du premier flux de gaz appauvri en CO₂ (13) en au moins un premier flux riche en hydrogène (14), et au moins un premier flux de gaz résiduaire (15).

17. Procédé selon la revendication 16, dans lequel ledit procédé comprend en outre : l'introduction d'au moins une première partie (15a) du premier flux de gaz résiduaire (15) dans la section de reformage (A) à titre d'alimentation supplémentaire comprenant du carbone.

18. Procédé selon l'une quelconque des revendications 16 ou 17, dans lequel ledit procédé comprend la fourniture d'au moins une première partie (14b) du flux riche en hydrogène (14) à la section de synthèse de méthanol (C), de préférence en mélange (6) avec le deuxième flux de gaz de synthèse (5).

19. Procédé selon l'une quelconque des revendications 16 à 18, dans lequel le système comprend en outre un système d'alimentation en combustible (H) et dans lequel une deuxième partie (15b) du premier flux de gaz résiduaire (15) et/ou une deuxième partie (14c) du flux riche en hydrogène (14) est fournie comme alimentation en combustible au système de combustible (H) et/ou à une chaudière à vapeur auxiliaire et/ou à une turbine à gaz.

20. Le procédé selon l'une quelconque des revendications 14-19, dans lequel le système comprend en outre un système d'alimentation en combustible (H), et dans lequel au moins une partie du premier flux de gaz appauvri en CO₂ (13) est séparée en au moins un deuxième flux de gaz appauvri en CO₂ (22) et au moins un troisième flux de gaz appauvri en CO₂ (23), et dans lequel au moins une partie dudit deuxième flux de gaz appauvri en CO₂ (22) est fournie comme alimentation en combustible audit système de combustible (H).

21. Procédé selon l'une quelconque des revendications 14-15, dans lequel au moins une partie du premier flux de gaz appauvri en CO₂ (13) est séparée en au moins un deuxième flux de gaz appauvri en CO₂ (28) et au moins un troisième flux de gaz appauvri en CO₂ (29), et dans lequel au moins une partie dudit deuxième flux de gaz appauvri en CO₂ (28) est fournie à la section de synthèse de méthanol (C), de préférence en mélange (6) avec le deuxième flux de gaz de synthèse (5).

22. Procédé selon l'une quelconque des revendications 14 à 21, comprenant l'étape de : fourniture du troisième flux de gaz de synthèse (10) provenant dudit train de refroidissement (B) à une température supérieure à celle du deuxième flux de gaz de synthèse (5, 6), de préférence dans laquelle la température du troisième flux de gaz de synthèse (10) est comprise entre 10 °C au-dessus du point de rosée du troisième flux de gaz de synthèse (10) et 360 °C.

23. Procédé selon l'une quelconque des revendications 14 à 22, dans lequel ledit flux de vapeur (2b) est mélangé avec le flux de gaz de purge (9) et/ou le troisième flux de gaz de synthèse (10), et/ou le premier flux de gaz flash (20) et/ou le flux de gaz résiduaire (21) de ladite section de distillation (D) avant d'être introduit dans la section de conversion (E).

24. Procédé selon l'une quelconque des revendications 14 à 23, dans lequel la teneur en carbone des gaz de combustion combinés (19) provenant de la combustion d'une partie de ladite deuxième partie (15b) du premier flux de gaz résiduaire (15) et/ou d'une partie d'une deuxième partie (14c) du flux riche en hydrogène (14) et du combustible à base d'hydrocarbures (18) vers le système est inférieure à 3% de la somme de la teneur en carbone dans l'alimentation en hydrocarbures (1) et le combustible à base d'hydrocarbures (18).

25. Installation comprenant le système selon l'une quelconque des revendications 1 à 13.
